# EUROPEAN PATENT APPLICATION

(11) **EP 4 140 517 A1**
(43) Date of publication of application: **01.03.2023**
(21) Application number: 21020434.3
(22) Date of filing: 31.08.2021
(51) Int. Cl.: A61M 1/36

(54) **EXTRACORPOREAL BLOOD CIRCUIT FOR HEPARIN-FREE HAEMODIALYSIS**

(71) Applicant: LundaTec AB, 223 63 Lund (SE)
(72) Inventor: LundaTec AB, 223 63 Lund (SE)

(57) **Abstract**

This utility model provides a Disposable Extracorporeal Blood Circuit for Heparin-free Haemodialysis. It is composed of the main line, clamps, two venous chambers and the line etc. One end of the main line and the line is designed in the shape of Y, and each of the two branches is connected with a venous chamber, forming a parallel circuit. The utility model is reasonably designed by adding another venous chamber, which is maintained parallel with the existing one. In the case of sudden looseness and falling off of the blood clots in the venous chamber, causing a sudden blockage, by using this extracorporeal blood circuit for heparin-free haemodialysis, it will not be necessary to be forced to discard the patient's blood. The blood will be transfused back into the patient's body quickly and safely, which will reduce the loss of patient's blood and the chances for medical disputes as well. Moreover, the heparin-free haemodialysis can be continued according to treatment needs, without the need of replacing the whole set of extracorporeal blood circuit so that waste of consumables will be reduced and the use-cost will be lowered.

## Description

### Technical Field

This utility model is a medical device and relates to the extracorporeal blood circuit, in particular to a kind of extracorporeal blood circuit used for heparin-free haemodialysis.

### Background

During haemodialysis, the patient's blood is connected to the vascular access, the disposable blood circuit, and the dialyzer, and heparin is normally used as the anticoagulant. For patients with bleeding tendency, heparin-free dialysis is adopted to avoid bleeding, but the major complication of heparin-free dialysis is blood coagulation, which will not only affect the dialysis effect but will also cause loss of patient's blood.

When performing the heparin-free haemodialysis, the body's thrombin system will be triggered, which will cause blood coagulation in the dialyzer and the blood circuit lines. In particular, when the blood clots in the venous chamber suddenly loosen and fall off, causing a blockage in the venous chamber, the blood circulation will be forced to stop and the blood can not be transfused back immediately, which will result in subsequent blood coagulation in the lines outside the venous chamber and in the dialyzer, further leading to the loss of patient's blood, aggravation of the patient's conditions and threats to the patient's life.

At present, during heparin-free haemodialysis, we normally flush the circuit with normal saline at a specified amount within a specified time to observe whether there will be blood clots in the dialyzer and the circuit. If there are big blood clots in the venous chamber, the blood will be transfused back, and the circuit will be replaced to continue the dialysis. However, in clinical practice, it is found that even small blood clots, in the case of sudden falling off, may also cause blockage that prevents the blood from being transfused back.

Blood coagulation during heparin-free haemodialysis usually occurs in the arterial chamber, the dialyzer and the venous chamber. Generally, sudden blockage that prevents the transfusing back of blood will not occur in the arterial chamber or the dialyzer, however, once it happens in the venous chamber, the patient's blood will not be transfused back to the body in a quick and safe way and the dialysis will have to be discontinued. In clinical practice, it is often forced to discard the patient's blood to end the treatment, which will lead to loss of patient's blood and the risk of aggravating the patient's condition.

To sum up, the current major deficiencies in performing heparin-free haemodialysis are: the blood circulation can not be established quickly and safely to transfuse the patient's blood back in the case that the blood clots in the venous chamber suddenly loosen and fall off, and cause a blockage in the venous chamber; the haemodialysis can not be resumed quickly; it is not possible to continue the heparin-free dialysis while reducing consumables; All the deficiencies will not only result in a rise in treatment cost, loss of patient's blood and medical disputes, but will even also lead to threats to the patient's life safety.

### Disclosure of Invention

The utility model is designed to provide the Disposable Extracorporeal Blood Circuit for Heparin-free Haemodialysis, a kind of single-use blood circuit used for heparin-free haemodialysis, which is simple, convenient and safe, and mainly aims to solve the problem of the existing disposable blood circuit during heparin-free haemodialysis that the blood circulation can not recover quickly when blockage occurs in the venous chamber.

The utility model, the Disposable Extracorporeal Blood Circuit for Heparin-free Haemodialysis is composed of the main line, the first clamp, the second clamp, the third clamp, the fourth clamp, the first venous chamber, the second venous chamber, the small clamp, the line ended with a venous pressure sensor, the branch line with a
small clamp and the line. One end of the main line is in the shape of Y that is respectively connected to the first venous chamber and the second venous chamber, and one end of the line is also in a Y shape that is connected to the other ends of the first venous chamber and the second venous chamber so as to form a parallel circuit; the first clamp is between the main line and the first venous chamber, the second clamp is between the main line and the second venous chamber, the third clamp is between the first venous chamber and the line, and the fourth clamp is between the second venous chamber and the line; the first venous chamber and the second venous chamber are respectively designed with a line equipped with a small clamp and ended with a venous pressure sensor and a branch line equipped with a small clamp.

The Disposable Extracorporeal Blood Circuit for Heparin-free Haemodialysis is placed into the packaging bag as a whole and EO sterilized after sealing.

The utility model makes improvement based on the existing disposable blood circuit for haemodialysis by adding another venous chamber to the original structure of the blood circuit and keeping it parallel with the existing chamber. The inlets and outlets of the two venous chambers are respectively connected to the lines at each end and are equipped with clamps as well. The utility model of extracorporeal blood circuit for heparin-free haemodialysis will quickly recover the smooth blood flow when blockage of blood clots occurs in the venous chamber during the heparin-free haemodialysis to transfuse the patient's blood back to the body and continue the dialysis treatment. During heparin-free haemodialysis, the establishment of a fast, safe and unobstructed extracorporeal blood circuit is an important premise to ensure the safe treatment of the patient.

The beneficial effects of the utility model are: (1) The utility model is properly designed. During heparin-free haemodialysis, when blood clots are observed in the venous chamber, or the blood clots suddenly loosen and fall off, causing a blockage in the venous chamber, immediately occlude the clamps at both ends of the venous chamber in use and open the back-up chamber, in this way, patient's blood will not have to be discarded, the dialysis treatment can be continued and the chances for medical disputes will be reduced. (2) There will be no
need to open a new set of extracorporeal blood circuit for haemodialysis. Cost accounting proves that it will be more economical by lowering the use cost.

### Brief Description of Drawings

Drawing 1 is the structural diagram of the disposable venous line specially used for heparin-free haemodialysis with two paralleled venous chambers
Drawing 2 is the structural diagram of the disposable venous line for haemodialysis made with existing technology

### Models for Carrying out the Invention

The utility model is further illustrated by the attached drawings and the models for carrying out the invention.

### Model 1

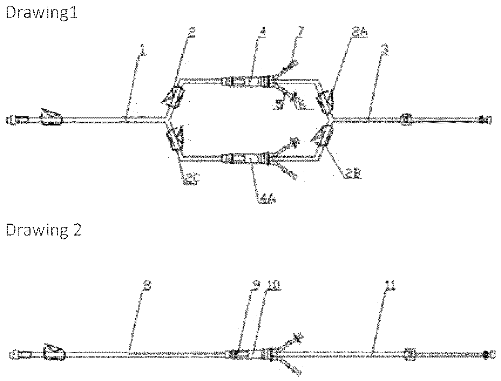

Refer to Drawing 1, the utility model of Disposable Extracorporeal Blood Circuit for Heparin-free Haemodialysis is composed of the main line (1), the first clamp (2), the second clamp (2C), the third clamp (2A),the fourth clamp (2B), the first venous chamber (4), the second venous chamber(4A), the small clamp(5), the line ended with a venous pressure sensor (6), the branch line (7) with a small clamp and the line(3). One end of the main line (1) is in the shape of Y that is respectively connected to the first venous chamber (4) and the second venous chamber (4A), and one end of the line (3) is also in a Y shape that is connected to the other ends of the first venous chamber (4) and the second venous chamber (4A), forming a parallel circuit; the first clamp (2) is between the main line (1) and the first venous chamber (4), the second clamp (2C) is between the main line (1) and the second venous chamber (4A), the third clamp (2A) is between the first venous chamber (4) and the line (3), and the fourth clamp (2B) is located between the second venous chamber (4A) and the line (3); the first venous chamber (4) and the second venous chamber (4A) are respectively designed with a line (6) equipped with a small clamp(5) and ended with a venous pressure sensor and a branch line (7) equipped with a small clamp.

The utility model
makes improvement based on the existing disposable blood circuit for haemodialysis. It maintains the original structure and components of the arterial line, the plastic bag for waster fluid, and the integrated infusion set while adding this utility model of venous line that is specially used for heparin-free haemodialysis with two paralleled venous chambers. The extracorporeal blood circuit is placed into the packaging bag as a whole and gets EO sterilized after sealing.

### Model 2

Refer to Drawing 2 which is a schematic diagram of the existing blood circuit. During the current heparin-free haemodialysis treatment, normal saline is usually used to flush the lines. If big blood clots are observed in the venous chamber (10) in use and the clots will cause blockage of the filter (9) in case of falling off, we will transfuse the patient's blood back and replace the whole set of blood circuit for haemodialysis to resume the dialysis treatment; if the blood clots in the venous chamber (10) suddenly loosen and fall off during the dialysis, blocking the filter (9), and the high venous pressure indicates a blockage in the venous chamber (10), the blood circulation will be forced to stop and the blood can not be transfused back immediately, which will cause blood coagulation outside the venous chamber in the lines and the dialyzer. In such cases, the only solution is to discard the dialyzer, the blood circuit for dialysis and the blood to end the treatment, or resume the treatment by using a new dialyzer and a new set of blood circuit, which will cause loss of patient blood and waste of consumables.

### Model 3

Refer to Drawing 1, the utility model is equipped in parallel with two identical venous chambers and it does not matter which one of them is used first. After the dialyzer and lines are properly connected, occlude the first clamp (2) and the third clamp (2A), install the second venous chamber (4A) to the dialyzer, open the fourth clamp (2B) and the second clamp (2C), then carry out the normal preflush procedure. When the second venous chamber (4A) is filled with the preflush fluid and reaches a certain preflush volume, occlude the fourth clamp (2B) and the second clamp (2C) to keep it on standby. Take the venous chamber (4A) off the dialyzer and have the first venous
chamber (4) installed, then open the first clamp (2A) and the third clamp (2). It will be ready when the venous chamber (4) is filled with preflush fluid and reaches a certain preflush volume. If it is necessary, the branch line (7) with a small clamp can be used to adjust the fluid level in the venous chamber.

When the blood clots in the venous chamber in use during dialysis suddenly loosen and fall off, causing blockage in the filter and high venous pressure, the utility model of venous line of two parallel venous chambers works as follows: immediately turn off the blood pump, occlude the first clamp (2) and the third clamp (2A) of the venous chamber (4) in use, occlude the small clamp (5) simultaneously, remove the venous pressure monitor line (6), assemble the second venous chamber (4A) on standby, open the connected small clamp (5) to monitor the venous pressure, then open the fourth clamp (2B) and the second clamp (2C), disconnect the main line (1) from the patient's vascular access, and check for blood clots: if there is no blood clot, reconnect and reduce the blood flow rate to 100ml/min, start the blood pump, observe the change in venous pressure, continue heparin-free dialysis or stop dialysis and return blood; if there is a blood clot, reduce the blood flow rate to 100ml/min, start the blood pump and discard the blood in the venous line of the main line (1) until the saline in the second venous chamber (4A) reaches the end to ensure that there is no blood clot, reconnect the end of the main line (1) to the patient's functioning vascular access, observe the change in venous pressure, and decide to continue heparin-free dialysis or stop dialysis and return blood according to the clinical needs.

## Claims

1. The Disposable Extracorporeal Blood Circuit for Heparin-free Haemodialysis is featured by its composition of the main line (1), the first clamp (2), the second clamp (2C), the third clamp (2A),the fourth clamp (2B), the first venous chamber (4), the second venous chamber(4A), the small clamp(5), the line ended with a venous pressure sensor(6), the branch line (7) with a small clamp and the line(3), among which, one end of the main line (1) is in the shape of Y which is respectively connected to the first venous chamber (4) and the second venous chamber (4A), and one end of the line (3) is also in the shape of Y that is connected to the other ends of the first venous chamber (4) and the second venous chamber (4A), forming a parallel circuit; the first clamp (2) is between the main line (1) and the first venous chamber (4), the second clamp (2C) is between the main line (1) and the second venous chamber (4A), the third clamp (2A) is between the first venous chamber (4) and the line (3) and the fourth clamp (2B) is between the second venous chamber (4A) and the line (3); the first venous chamber (4) and the second venous chamber (4A) are respectively designed with a line equipped with a small clamp and ended with a venous pressure sensor (6) and a branch line (7) equipped with a small clamp.

2. The feature of the Disposable Extracorporeal Blood Circuit for Heparin-free Haemodialysis specified in (1) of the Patent Claims is that the blood circuit is placed into the packaging bag as a whole and EO sterilized after sealing.
